# EUROPEAN PATENT SPECIFICATION

(11) **EP 3 230 420 B1**
(45) Date of publication and mention of the grant of the patent: **07.04.2021**
(21) Application number: 15804830.6
(22) Date of filing: 07.12.2015
(51) Int. Cl.: C10M 169/04, C10N 30/06, C10N 40/20

(54) **LUBRICATING OIL COMPOSITION FOR SLIDING GLIDE SURFACE**
SCHMIERÖLZUSAMMENSETZUNG FÜR VERSCHIEBBARE GLEITOBERFLÄCHEN
COMPOSITION D'HUILE LUBRIFIANTE POUR SURFACE DE GUIDAGE COULISSANTE

(30) Priority: 09.12.2014 JP 2014249031
(43) Date of publication of application: 18.10.2017
(73) Proprietor: Shell Internationale Research Maatschappij B.V., 2596 HR The Hague (NL)
(72) Inventor: NAGAKARI, Mitsuhiro, Minato-ku Tokyo 135-8074 (JP); NAITOU, Ayano, Kanagawa 243-0303 (JP)
(74) Representative: Shell Legal Services IP
(86) International application number: PCT/EP2015/078784
(87) International publication number: WO 2016/091786

(56) References cited:
- JP-A- 2004 182 790
- JP-A- 2005 068 370
- US-A- 4 634 543
- US-A1- 2009 062 166

## Description

### Field of the Invention

The present invention relates to a lubricating oil composition which is suitable for lubricating a sliding guide surface of a machine tool or the like.

### Background of the Invention

In order to carry out high precision machining by means of a machine tool, it is essential for the positioning accuracy of a feed shaft of the machine tool to be excellent, with micron level precision being required in some cases. However, lubricating oils are used because positioning accuracy can deteriorate due to friction resistance produced on a guide surface of a machine tool having a sliding guide surface, and it is necessary for a lubricating oil used on this guide surface to exhibit low friction.

In addition, lubricating oils used in machine tools can also be used to lubricate gears and the like in addition to guide surfaces as described above, and in such cases load bearing properties are also required as an important feature.

US4634543 describes a fluid composition for use in a shock absorber, comprising (a) a lubricating base oil, (b) 10 to 1,000 ppm, as calculated as boron, of a boron-containing compound, and (c) 100 to 3,000 ppm, as calculated as phosphorus, of phosphoric acid ester and/or phosphorous acid ester.

JP2005068370 is directed to a lubricating oil composition containing a reaction product of a polyalkylene polyamine, a straight or branched chain saturated or unsaturated univalent fatty acid and an alkenyl-substituted succinic acid or its anhydride, and a straight or branched chain saturated or unsaturated univalent fatty acid. Said composition is taught as useful for a lubricant for ATF exhibiting a high transmission torque capacity and high shudder preventive durability.

JP2004182790 teaches a lubricating oil composition for a sliding guide surface which comprises at least one kind of a base oil selected from a mineral oil, oils and fats and a synthetic oil, at least one kind selected from a phosphorus compound and a sulfur compound and a specific polyalkylene glycol derivative.

US2009/062166 describes a slideway lubricant composition comprising a sufficient amount of an isomerized base oil having consecutive numbers of carbon atoms and has less than 10 wt % naphthenic carbon by n-d-M for the composition to separate from water in less than 60 minutes at 54° C. as measured according to ASTM D-1401-2002.

Therefore, because smooth movement and high precision are required of guide surfaces, a variety of friction-reducing agents are blended in lubricating oils used on guide surfaces in order to reduce friction. For example, JP 11-505283 discloses that attempts have been made to achieve low friction properties and good sliding properties by using combinations of acidic esters of phosphoric acid and phosphonic acid esters.

### Summary of Invention

Conventional lubricating oil compositions have yet to achieve satisfactory lubricating properties for machine tools for which high precision machining is required, and an objective of the present invention, which has been devised with these circumstances in mind, is to obtain a lubricating oil composition having further improved frictional properties and extreme pressure properties.

As a result of various investigations and research carried out with the aim of reducing friction and achieving good extreme pressure properties, as described above, it was found that in cases where a combination of a secondary phosphite and a fatty acid was used, a lower coefficient of friction and higher load bearing properties could be achieved than in cases where either of these additives was used in isolation, and the present invention was completed on the basis of these findings.

The present invention provides a lubricating oil composition for a sliding guide surface, which contains a base oil belonging to group III in the API (American Petroleum Institute) base oil categories, a secondary phosphite represented by formula 1 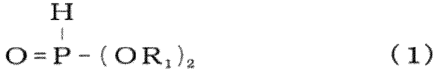
wherein R₁ denotes a saturated or unsaturated alkyl group having 9-18 carbon atoms, and
and a fatty acid represented by formula 2

   R₂COOH (2)

   wherein R₂ denotes a saturated or unsaturated alkyl group having 9-17 carbon atoms, wherein the secondary phosphite is contained at a quantity of between 0.05 mass% and 3 mass% relative to the overall quantity of the composition, and the fatty acid is contained at a quantity of between 0.01 mass% and 2 mass% relative to the overall quantity of the composition.The lubricating oil composition of the present invention can exhibit excellent frictional properties and load bearing properties on a sliding guide surface of a machine tool or the like, and can be effectively used as a lubricating oil composition for a sliding guide surface.

### Detailed Description of the Invention

Examples of group III base oils include a paraffin-based mineral oil produced by subjecting a lubricating oil distillate, which is obtained by subjecting crude oil to atmospheric distillation, to a high degree of hydrorefining, a base oil obtained by refining a wax, which is produced in a dewaxing process, using an isodewax process in which conversion and dewaxing are carried out, or a base oil that has been refined using the wax isomerization process used by Mobil Oil.

The viscosity of these group III base oils is not particularly limited, but the viscosity index should be from 120 to 180, and preferably from 130 to 150. The kinematic viscosity at 40°C is preferably from 2 to 680 mm²/s, and more preferably from 8 to 220 mm²/s. In addition, the total sulfur content is suitably 300ppm or less, and preferably 10ppm or less. The total nitrogen content is suitably 10ppm or less, and preferably 1ppm or less. Furthermore, the aniline point is suitably from 80 to 150°C, and preferably from 110 to 135°C.

In addition, as a base oil belonging to group III, a GTL (gas to liquid) base oil synthesized by the Fischer-Tropsch process, which is a technique for converting natural gas into liquid fuel, has a significantly lower sulfur content and aromatics content and a significantly higher paraffin proportion than a mineral oil-based base oil refined from crude oil, and therefore exhibits excellent oxidation stability and extremely low evaporative losses, and can be advantageously used as the base oil in the present invention.

The viscosity properties of this GTL base oil are not particularly limited, but the viscosity index is generally from 130 to 180, and more preferably from 140 to 175. In addition, the kinematic viscosity at 40°C is suitably from 2 to 680 mm²/s, and more preferably from 5 to 120 mm²/s. In addition, the total sulfur content is generally less than 10ppm, and the total nitrogen content is generally less than 1ppm. An example of this type of GTL base oil product is SHELL XHVI™.

The secondary phosphite mentioned above is represented by formula 1 below. 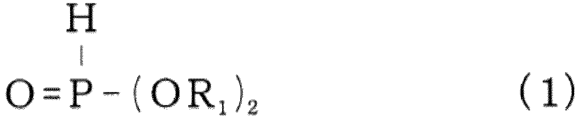 In formula 1 above, R₁ is a saturated or unsaturated alkyl group having 9-18 carbon atoms. This alkyl group is often linear, but may be branched.

Examples of this type of secondary phosphite include dinonyl hydrogen phosphite, didecyl hydrogen phosphite, diundecyl hydrogen phosphite, didodecyl hydrogen phosphite (dilauryl hydrogen phosphite), ditridecyl hydrogen phosphite, ditetradecyl hydrogen phosphite (dimyristyl hydrogen phosphite), dipentadecyl hydrogen phosphite, dihexadecyl hydrogen phosphite (dipalmityl hydrogen phosphite), diheptadecyl hydrogen phosphite, dioctadecyl hydrogen phosphite (distearyl hydrogen phosphite), dioleyl hydrogen phosphite, dilinoleyl hydrogen phosphite and dilinolenyl hydrogen phosphite.

This type of secondary phosphite is used at a quantity of the order of between 0.05 mass% and 3 mass%, and preferably between 0.1 mass% and 2.5 mass%, relative to the overall quantity of the lubricating oil composition.

The fatty acid is represented by formula 2 below.

R₂COOH (2)

In formula 2 above, R₂ is a saturated or unsaturated alkyl group having 9-17 carbon atoms.

Examples of this type of fatty acid include capric acid, lauric acid, myristic acid, palmitic acid, stearic acid, isostearic acid, oleic acid, linoleic acid and linolenic acid.

This type of fatty acid is used at a quantity of the order of between 0.01 mass% and 2 mass%, and preferably between 0.02 mass% and 1.5 mass%, relative to the overall quantity of the lubricating oil composition.

Metal deactivators, anti-wear agents, and the like, can also be added to this lubricating oil composition. Examples of metal deactivators include thiadiazole derivatives, for example 2,5-bis(alkyldithio)-1,3,4-thiadiazole compounds such as 2,5-bis(heptyldithio)-1,3,4-thiadiazole, 2,5-bis(nonyldithio)-1,3,4-thiadiazole, 2,5-bis(dodecyldithio)-1,3,4-thiadiazole and 2,5-bis(octadecyldithio)-1,3,4-thiadiazole; 2,5-bis(N,N-dialkyldithiocarbamyl)-1,3,4-thiadiazole compounds such as 2,5-bis(N,N-diethyldithiocarbamyl)-1,3,4-thiadiazole, 2,5-bis(N,N-dibutyldithiocarbamyl)-1,3,4-thiadiazole and 2,5-bis(N,N-dioctyldithiocarbamyl)-1,3,4-thiadiazole; and 2-N,N-dialkyldithiocarbamyl-5-mercapto-1,3,4-thiadiazole compounds such as 2-N,N-dibutyldithiocarbamyl-5-mercapto-1,3,4-thiadiazole and 2-N,N-dioctyldithiocarbamyl-5-mercapto-1,3,4-thiadiazole. In some cases, it is possible to use a benzotriazole or benzotriazole derivative, a benzimidazole or benzimidazole derivative, an imidazole or imidazole derivative, a benzothiazole or benzothiazole derivative, a benzoxazole derivative, a triazole derivative, or the like. It is possible to use one or more of these metal deactivators at a quantity of approximately 0.01-0.5 mass% in the lubricating oil composition.

Examples of the anti-wear agent include diisobutyl disulfide, diisobutyl trisulfide, di-t-butyl trisulfide, dioctyl trisulfide, di-t-nonyl trisulfide, di-t-benzyl trisulfide, and other polysulfides. It is also possible to use a sulfurized olefin, a sulfurized oil or fat, or the like. It is possible to use one or more of these sulfur-based anti-wear agents at a quantity of from approximately 0.1 to 3 mass% in the lubricating oil composition.

In addition, these metal deactivators and anti-wear agents can be used in isolation or in appropriate combinations thereof, and in cases where these are used in combination, a low coefficient of friction can be achieved, better abrasion resistance and extreme pressure properties can be achieved, and a sliding guide surface can be effectively lubricated under harsh conditions.

If necessary, antioxidants such as amine-based and phenol-based antioxidants, corrosion inhibitors, structure stabilizers, viscosity modifiers, dispersing agents, pour point depressants, anti-foaming agents and other known additives can be blended as appropriate in the lubricating oil composition of the present invention.

The viscosity grade of the lubricating oil composition for a sliding guide surface described above should be VG22 to VG220, and preferably VG32 to VG68, according to ISO viscosity grades.

The lubricating oil composition for a sliding guide surface of the present invention will now be described in specific terms through working examples and comparative examples, but the present invention is in no way limited to these examples.

### Examples

The following materials were prepared in order to produce the working examples and comparative examples.

### Base oils

Base oil 1: GTL (gas to liquid) base oil belonging to group III (properties: kinematic viscosity at 100°C: 7.579mm²/s, kinematic viscosity at 40°C: 43.69mm²/s, viscosity index (VI): 141, density at 15°C: 0.8284) (Shell XHVI-8 manufactured by Royal Dutch Shell)
Base oil 2: Refined mineral oil belonging to group III (properties: kinematic viscosity at 100°C: 7.545mm²/s, kinematic viscosity at 40°C: 45.50mm²/s, viscosity index (VI): 132, density at 15°C: 0.8453) (Yu-Base 8 manufactured by SK Innovation)

### Additives

Additive 1-1: Dilauryl hydrogen phosphite
Additive 1-2: Dioleyl hydrogen phosphite
Additive 2-1: Lauric acid
Additive 2-2: Stearic acid
Additive 2-3: Oleic acid
Additive 3: Dibutyl hydrogen phosphite
Additive 4: Di-2-ethylhexyl hydrogen phosphite
Additive 5: Diethyl benzylphosphonate
Additive 6: Caprylic acid
Additive 7: Thiadiazole
Additive 8: Di-t-dodecyl trisulfide

### Working Examples 1-8 and Comparative Examples 1-11

Lubricating oil compositions for a sliding guide surface of Working Examples 1-8 and Comparative Examples 1-11 were prepared using the materials mentioned above according to the compositions shown in Tables 1-3 below. The blending quantities of the components are shown as mass%.

### Tests

### Coefficient of friction: Pendulum type coefficient of friction test

The coefficients of friction of the lubricating oil compositions of Working Examples 1-8 and Comparative Examples 1-11 were measured using a Soda type pendulum type oiliness tester manufactured by Shinko Engineering Co., Ltd. In this test, a test oil was applied to a wear part that was the support point of a pendulum, the pendulum was made to swing, and the coefficient of friction was determined from the attenuation of the swing. The test was carried out at room temperature (25°C).

Evaluation of the test was carried out according to the following criteria:
A coefficient of friction of 0.110 or less was deemed to be ○ (pass).
A coefficient of friction of greater than 0.110 was deemed to be × (fail).

### Flash point

The flash points of samples of Working Examples 1-8 and Comparative Examples 1-11 were measured five times in accordance with JIS K2265-4 using a Cleveland open cup automatic flash point measurement apparatus, and the average value was determined by rounding off to 1 digit after the decimal point. The thermometer used was a no. 32 thermometer specified in JIS B7410 (COC).

Evaluation of the test was carried out according to the following criteria:
A flash point of 220°C or higher was deemed to be ○ (pass).
A flash point of less than 220°C was deemed to be × (fail) .

### Load bearing properties test: Shell four-ball EP test

Working Examples 1 and 12 and Comparative Examples 5 and 6 were subjected to a load bearing test in accordance with ASTM D2783.
Conditions: Speed of rotation: 1760 ± 40rpm
Duration: 10 seconds
Temperature: room temperature

Test items: ISL (Initial Seizure Load, units kgf) was obtained for Working Examples 1, 2 and 5, and for Comparative Examples 1 and 3-6; and WL (Weld Load, units kgf) was obtained for Working Examples 1 and 5, and for Comparative Example 4.

Test method: numerical values were determined by applying loads of 50 kgf, 63 kgf, 80 kgf, 100 kgf, 126 kgf, 160 kgf, 200 kgf and 250 kgf up to the WL.

Evaluation of the ISL was carried out according to the following criteria:
80kgf or more was deemed to be o (pass).
Less than 80kgf was deemed to be × (fail).

In addition, evaluation of the WL was carried out according to the following criteria:
126kgf or more was deemed to be ○ (pass).
Less than 126kgf was deemed to be × (fail).

### Abrasion resistance test: Shell four-ball wear test

The test equipment and test methods were such that a load of 40 kgf was applied in accordance with ASTM D4172, the oil temperature was 75°C, the tester was rotated at 1200 rpm for 1 hour, and the diameter of an abrasion mark occurring at the point of contact was measured. Working Examples 1 and 5 and Comparative Example 4 were subjected to this test.

Evaluation of the test was carried out according to the following criteria:
An abrasion mark diameter of 0.50 mm or less was deemed to be ○ (pass).
An abrasion mark diameter of greater than 0.50 mm was deemed to be × (fail).

### Storage stability

The lubricating oil compositions of Working Examples 1-8 and Comparative Examples 1-11 were allowed to stand for 1 day (24 hours) at 25°C, after which the presence/absence of cloudiness or precipitation was determined visually.

Examples in which cloudiness and precipitation had not occurred were deemed to be o (pass).

Examples in which cloudiness or precipitation had occurred are as shown in the tables.

With regard to storage stability, examples in which cloudiness or precipitation had occurred were unsuitable as lubricating oil compositions for sliding guide surfaces.

### Test results

The test results for the working examples and comparative examples are shown in Tables 1-3.

**Table 1**

| | Working Example 1 | Working Example 2 | Working Example 3 | Working Example 4 | Working Example 5 |
|---|---|---|---|---|---|
| Base oil 1 | 99.88 | 99.85 | 99.86 | 99.85 | 97 |
| Base oil 2 | | | | | |
| Additive 1-1 | 0.1 | 0.1 | 0.1 | 0.1 | 2 |
| Additive 1-2 | | | | | |
| Additive 3 | | | | | |
| Additive 4 | | | | | |
| Additive 5 | | | | | |
| Additive 6 | | | | | |
| Additive 2-1 | | | 0.04 | | |
| Additive 2-2 | 0.02 | 0.05 | | | 1 |
| Additive 2-3 | | | | 0.05 | |
| Additive 7 | | | | | |
| Additive 8 | | | | | |
| Coefficient of friction | 0.097 | 0.097 | 0.106 | 0.109 | 0.096 |
| Flash point (°C) | 262 | 266 | 268 | 270 | 262 |
| Four-ball EP: ISL | 80 | 100 | | | 126 |
| Four-ball EP: WL | 126 | | | | 160 |
| Four-ball wear | 0.48 | | | | 0.41 |
| Storage stability | ○ | ○ | ○ | ○ | ○ |

**Table 2**

| | Comparative Example 1 | Comparative Example 2 | Comparative Example 3 | Comparative Example 4 | Comparative Example 5 | Comparative Example 6 |
|---|---|---|---|---|---|---|
| Base oil 1 | 99.9 | 99.8 | 99.95 | 99.75 | 99.97 | 99.87 |
| Base oil 2 | | | | | | |
| Additive 1-1 | 0.1 | 0.2 | | | | 0.1 |
| Additive 1-2 | | | | | | |
| Additive 3 | | | | | | |
| Additive 4 | | | | | | |
| Additive 5 | | | | | | |
| Additive 6 | | | | | 0.03 | 0.03 |
| Additive 2-1 | | | | | | |
| Additive 2-2 | | | 0.05 | 0.25 | | |
| Additive 2-3 | | | | | | |
| Additive 7 | | | | | | |
| Additive 8 | | | | | | |
| Coefficient of friction | 0.146 | 0.122 | 0.101 | 0.092 | 0.125 | 0.118 |
| Flash point (°C) | 272 | 266 | 200 | 268 | 274 | 266 |
| Four-ball EP: ISL | 80 | | 50 | 63 | 50 | 100 |
| Four-ball EP: WL | | | | 126 | | |
| Four-ball wear | | | | 0.73 | | |
| Storage stability | ○ | ○ | ○ | ○ | ○ | ○ |

**Table 3**

| | Working Example 6 | Working Example 7 | Working Example 8 | Comparative Example 7 | Comparative Example 8 | Comparative Example 9 | Comparative Example 10 | Comparative Example 11 |
|---|---|---|---|---|---|---|---|---|
| Base oil 1 | 98.5 | 98.1 | | 98.9 | 98.9 | 99.95 | 99 | 98.1 |
| Base oil 2 | | | 98.5 | | | | | |
| Additive 1-1 | 1.2 | | 1.2 | | | | | |
| Additive 1-2 | | 1.6 | | | | | | |
| Additive 3 | | | | 0.8 | | | | |
| Additive 4 | | | | | 0.8 | | | |
| Additive 5 | | | | | | | | 0.6 |
| Additive 6 | | | | | | | | |
| Additive 2-1 | | | | | | | | |
| Additive 2-2 | 0.25 | 0.25 | 0.25 | 0.25 | 0.25 | | | 0.25 |
| Additive 2-3 | | | | | | | | |
| Additive 7 | 0.05 | 0.05 | 0.05 | 0.05 | 0.05 | 0.05 | | 0.05 |
| Additive 8 | | | | | | | 1 | 1 |
| Coefficient of friction | 0.097 | 0.095 | 0.096 | 0.114 | 0.115 | 0.146 | 0.143 | 0.122 |
| Flash point (°C) | 266 | 270 | 264 | 268 | 268 | 272 | 250 | 214 |
| Four-ball EP: ISL | | | | | | | | |
| Four-ball EP: WL | | | | | | | | |
| Four-ball wear | | | | | | | | |
| Storage stability | ○ | ○ | ○ | ○ | ○ | ○ | ○ | ○ |

As shown in Table 1, the composition of Working Example 1, which contained base oil 1 and additives 1-1 and 2-2, had a low coefficient of friction of 0.097 and a high flash point of 262°C, and therefore passed in terms of both and was found to be excellent as a lubricating oil composition for a sliding guide surface. However, the composition of Comparative Example 1, which did not contain additive 2-2, passed in terms of flash point, but was found to be unsuitable due to having a high coefficient of friction of 0.146. In addition, a composition such as that of Comparative Example 2 which did not contain additive 2-2 was undesirable because the coefficient of friction was unsuitable at 0.122, even though it contained twice the quantity of additive 1-1 compared with Comparative Example 1.

Although the composition of Working Example 2 contained a larger amount of additive 2-2 than that of Working Example 1, the coefficient of friction was the same at 0.097 and the flashpoint was slightly higher at 266°C, and was therefore excellent in the same way as in Working Example 1. On the other hand, a composition such as that of Comparative Example 3 which did not contain additive 1-1 passed in terms of coefficient of friction and flashpoint, but had an ISL in the Shell four-ball EP test of only 50kgf and was therefore deemed unsuitable. The composition of Comparative Example 4 had five times the amount of additive 2-2 in comparison with that of Comparative Example 3 and passed in terms of coefficient of friction and flashpoint. However, although Comparative Example 4 passed with a WL of 126kgf in the Shell four-ball EP test, the ISL was only 63kgf and the abrasion mark diameter in the Shell four-ball wear test was high (0.73 mm) and said composition was therefore considered unsuitable.

The composition of Working Example 3 contained additive 1-1 and additive 2-1 and passed in terms of coefficient of friction and flashpoint, and the composition of Working Example 4 which contained additive 1-1 and additive 2-3 also passed in terms of both coefficient of friction and flashpoint, and these compositions were therefore deemed suitable.

In addition, the composition in Working Example 5 contained a considerably larger amount of additive 1-1 (2 mass%) and additive 2-2 (1 mass%), but still passed in terms of coefficient of friction and flashpoint, exhibited high values for ISL (126kgf) and WL (160kgf) in the Shell four-ball EP test, and had a small value of 0.41 mm in the Shell four-ball wear test, and was therefore deemed suitable.

Meanwhile, the composition of Comparative Example 5 which employed additive 6 (caprylic acid) as the fatty acid passed in terms of flashpoint but had a large coefficient of friction and had an ISL of only 50kgf in the Shell four-ball EP test, and was therefore not considered suitable. In Comparative Example 6, the same amount of additive 1-1 as in Working Examples 1-4 was added to the composition of Comparative Example 5, and although the composition passed in terms of flashpoint, the coefficient of friction was high (0.118) and the composition was therefore not considered suitable.

In Working Example 6, the amount of additive 1-1 was increased to 1.2 mass% and the amount of additive 2-2 was increased to 0.25 mass% in comparison with Working Examples 1 and 2, and additive 7 was also added; the composition passed in terms of both coefficient of friction and flashpoint. In Working Example 7, 1.6 mass% of additive 1-2 was blended with the composition of Working Example 6 instead of additive 1-1, and this composition also passed in terms of both coefficient of friction and flashpoint. Furthermore, in the composition of Working Example 8, base oil 1 in the composition of Working Example 6 was changed to base oil 2, and this composition also passed in terms of coefficient of friction and flashpoint, so all of these compositions were deemed suitable as lubricating oil compositions for a sliding guide surface.

In contrast to this, the composition of Comparative Example 7 did not employ additive 1-2 (molecular weight 574) in Working Example 7, rather it employed half the amount (approximately 1.4 times that of Working Example 7 in terms of the number of moles) of additive 3 (molecular weight 194), and although it passed in terms of flashpoint, the coefficient of friction was large and therefore failed. In addition, the composition of Comparative Example 8 likewise employed half the amount (substantially the same amount as in Exemplary Embodiment 7 in terms of the number of moles) of additive 4 (molecular weight 306), and the composition passed in terms of flashpoint but the coefficient of friction was large and therefore failed; neither of these compositions achieved good results.

The composition of Comparative Example 9 did not employ either additive 1 or additive 2, and additive 7 alone was added thereto; the composition passed in terms of flashpoint but had a very large coefficient of friction and therefore failed in this regard. Furthermore, the composition of Comparative Example 10 did not employ either additive 1 or additive 2, and additive 8 alone was added thereto; the composition passed in terms of flashpoint but had a very large coefficient of friction and therefore failed in this regard; both of these compositions were deemed unsuitable as lubricating oil compositions for a sliding guide surface.

The composition of Comparative Example 11 employed the same amount of additive 2-2 as in Working Examples 6-8 and also employed additive 5 without employing additive 1-1 or additive 1-2, and additives 7 and 8 were also added thereto; this composition had a large coefficient of friction of 0.122 and also had a low flashpoint of 214°C and therefore failed in terms of both; the composition was deemed unsuitable as a lubricating oil composition for a sliding guide surface.

It should be noted that in Working Example 1, the ISL in the Shell four-ball EP test was 80 kgf and the WL exhibited a high value of 126 kgf, while the value in the Shell four-ball wear test was also small at 0.48 mm and therefore good results were exhibited. Furthermore, in Working Example 5, the ISL in the Shell four-ball EP test was 126 kgf and the WL exhibited a high value of 160 kgf, while the value in the Shell four-ball wear test was also small at 0.41 mm and therefore good results were exhibited.

In addition, no cloudiness or precipitation was produced in any of Working Examples 1-8 or Comparative Examples 1-11 and therefore these were all deemed to pass.

## Claims

1. A lubricating oil composition for a sliding guide surface, which contains a base oil belonging to group III in the API base oil categories, a secondary phosphite represented by formula 1 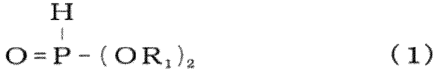
wherein R₁ denotes a saturated or unsaturated alkyl group having 9-18 carbon atoms, and
a fatty acid represented by formula 2
R₂COOH (2)
wherein R₂ denotes a saturated or unsaturated alkyl group having 9-17 carbon atoms, wherein the secondary phosphite is contained at a quantity of between 0.05 mass% and 3 mass% relative to the overall quantity of the composition, and the fatty acid is contained at a quantity of between 0.01 mass% and 2 mass% relative to the overall quantity of the composition.

2. A lubricating oil composition for a sliding guide surface according to claim 1, which further contains an anti-wear agent and/or a metal deactivator.

3. Use of a lubricating oil composition according to any preceding claim for lubricating a sliding guide surface of a machine tool.

## Patentansprüche

1. Schmierölzusammensetzung für eine Gleitführungsoberfläche, die Folgendes enthält: ein Grundöl, das zu Gruppe III in den API-Grundölkategorien gehört, ein sekundäres Phosphit, das durch Formel 1 dargestellt ist, 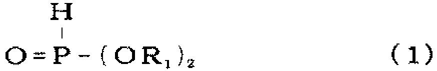 wobei R₁ eine gesättigte oder ungesättigte Alkylgruppe bezeichnet, die 9-18 Kohlenstoffatome aufweist, und eine Fettsäure, die durch Formel 2 dargestellt ist,
R₂COOH (2)
wobei R₂ eine gesättigte oder ungesättigte Alkylgruppe bezeichnet, die 9-17 Kohlenstoffatome aufweist, wobei das sekundäre Phosphit in einer Menge zwischen 0,05 Massen-% und 3 Massen-% relativ zu der Gesamtmenge der Zusammensetzung enthalten ist, und die Fettsäure in einer Menge zwischen 0,01 Massen-% und 2 Massen-% relativ zu der Gesamtmenge der Zusammensetzung enthalten ist.

2. Schmierölzusammensetzung für eine Gleitführungsoberfläche nach Anspruch 1, die ferner ein Verschleißschutzmittel und/oder einen Metalldeaktivator enthält.

3. Verwendung einer Schmierölzusammensetzung nach einem der vorhergehenden Ansprüche zum Schmieren einer Gleitführungsoberfläche einer Werkzeugmaschine.

## Revendications

1. Composition d'huile lubrifiante pour une surface de guidage coulissante, qui contient une huile de base appartenant au groupe III dans les catégories d'huiles de base API, un phosphite secondaire représenté par la formule 1 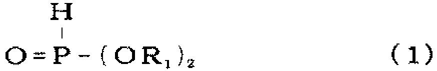 dans laquelle R₁ désigne un groupe alkyle saturé ou insaturé ayant 9 à 18 atomes de carbone, et un acide gras représenté par la formule 2
R₂COOH (2)
dans laquelle R₂ désigne un groupe alkyle saturé ou insaturé ayant 9 à 17 atomes de carbone, le phosphite secondaire étant contenu en une quantité comprise entre 0,05 % en masse et 3 % en masse par rapport à la quantité totale de la composition, et l'acide gras étant contenu en une quantité comprise entre 0,01 % en masse et 2 % en masse par rapport à la quantité globale de la composition.

2. Composition d'huile lubrifiante pour une surface de guidage coulissante selon la revendication 1, qui contient en outre un agent anti-usure et/ou un désactivateur de métal.

3. Utilisation d'une composition d'huile lubrifiante selon l'une quelconque des revendications précédentes pour lubrifier une surface de guidage coulissante d'une machine-outil.
